(19) Europäisches Patentamt
European Patent Office
Office européen des brevets

(11) Veröffentlichungsnummer: **0 378 089 B1**

# (12) EUROPÄISCHE PATENTSCHRIFT

(45) Veröffentlichungstag der Patentschrift: **29.06.94**

(51) Int. Cl.$^{5}$: **C07D 239/47**

(21) Anmeldenummer: **90100075.2**

(22) Anmeldetag: **03.01.90**

## (54) Verfahren zur Herstellung von 2-Amino-4-fluorpyrimidinderivaten.

(30) Priorität: **10.01.89 DE 3900471**

(43) Veröffentlichungstag der Anmeldung:
**18.07.90 Patentblatt 90/29**

(45) Bekanntmachung des Hinweises auf die Patenterteilung:
**29.06.94 Patentblatt 94/26**

(84) Benannte Vertragsstaaten:
**BE CH DE FR GB IT LI NL**

(56) Entgegenhaltungen:

**CHEMICAL ABSTRACT, Band 86, Nr.25, 20. Juni 1977, Columbus, Ohio, USA STUDENTSOV, E.P. et al. "6-Fluoroisocytosine" Seite 620, Spalte 2, Zusammenfassung-Nr. 190 000w & Otkrytiya, Izobret., Prom. Obraztsy, Tovarnye Znaki 1977 54(7), 68**

**CHEMICAL ABSTRACTS, Band 59, Nr. 12, 9. Dezember 1963, Columbus, Ohio, USA IRIS WEMPEN et al. "The synthesis of 6-fluorocytosine and related compounds" Spalte 15 274, Zusammenfassung-Nr. 15 274b & J. Med. Chem. 6 (6), 688-93 (1963)**

(73) Patentinhaber: **BASF Aktiengesellschaft
Carl-Bosch-Strasse 38
D-67063 Ludwigshafen(DE)**

(72) Erfinder: **Hamprecht, Gerhard, Dr.
Rote-Turm-Strasse 28
D-6940 Weinheim(DE)**

**EP 0 378 089 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 73, Nr. 5, 3. August 1970, Columbus, Ohio, USA BANKS RONALD E. et al. "Heterocyclic polyfluoro-compounds. XIX. Synthesis of and nucleophilic substitution in some 2,4,6-trifluoropyri- midines: formation of tri- fluoromethylpyrimidines by pyrolysis of tetrafluoropyri- midine" Seite 361, Spalte 2, Zusammenfassung-Nr. 25 402c & J. Chem. Soc., C 1970, (9), 1280-5

CHEMICAL ABSTRACTS, Band 85, Nr. 11, 13. September 1976, Columbus, Ohio, USA SHKURKO O.P. et al. "Effect of the medium on the reaction of 2,4-difluoro- and 2,4,6-trifluoropyrimidines with ammonia" Seite 470, Spalte 1, Zusammenfassung -Nr. 77 134j & Kratk. Tezisy - Vses. Soveshch. Probl. Mekh. Geteroliticheskikh Reakts. 1974, 46-7

CHEMICAL ABSTRACTS, Band 80, Nr. 11, 18. März 1974, Columbus, Ohio, USA SHKURKO, O.P. et al. "Pyri- midines. XLII. Synthesis of 2- and 6-substituted 4-fluoropyrimidines" Seite 356, Spalte 2, Zusammenfassung-Nr. 59 913e & Izv. Sib. Otd. Akad. Nauk SSSR, Ser. Khim. Nauk 1973, (6), 81-5

CHEMICAL ABSTRACTS, Band 80, Nr. 3, 21. Jänner 1974, Columbus, Ohio, USA SHKURKO, O.P. et al. "Pyri- midines. XLI. Reaction of 2,4-difluoro- and 2,4,6- trifluoropyrimidines with ammonia in various solvents" Seite 387, Spalte 2, Zusammenfassung-Nr 14 409d & Izv. Sib. Otd. Akad. Nauk SSSR, Ser. Khim. Nauk 1973, (5), 104-8

Liebigs Ammalen der Chemie 1983; Heft 5; S. 721-43

**Beschreibung**

Die vorliegende Erfindung betrifft ein verbessertes Verfahren zur Herstellung von 2-Amino-4-fluorpyrimidinderivaten der allgemeinen Formel I

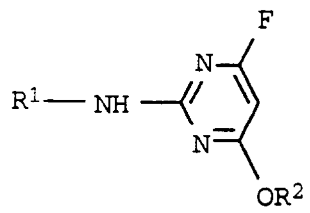

I

in der die Reste folgende Bedeutung haben:

$R^1$ Wasserstoff, Alkyl, ausgewählt aus der Gruppe Methyl, Ethyl, Propyl, 1-Methylethyl, n-Butyl, 1-Methylpropyl, 2-Methylpropyl und 1,1-Dimethylethyl sowie n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 2-Methylhexyl, 3-Methylhexyl, 4-Methylhexyl, 5-Methylhexyl, 1-Ethylpentyl, 2-Ethylpentyl, 1-Propylbutyl und Octyl, Alkenyl, ausgewählt aus der Gruppe Allyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1,2-Dimethyl-2-propenyl, 1,1-Dimethyl-2-propenyl, 1-Methyl-3-butenyl, 1-Ethyl-2-propenyl, 2-Hexenyl, 1-Methyl-2-pentenyl, 1-Ethyl-2-butenyl und 2-Ethyl-2-butenyl, Alkinyl, ausgewählt aus der Gruppe 2-Propinyl, 2-Butinyl, 3-Butinyl, 1-Methyl-2-propinyl, 1-Methyl-2-butinyl, 1-Ethyl-2-propinyl, 2-Pentinyl, 3-Pentinyl, 4-Pentinyl und 1-Methyl-2-pentinyl, $C_3$-$C_8$-Cycloalkyl, ein Phenylrest oder ein Benzylrest,

$R^2$ einer der Reste $R^1$ mit Ausnahme von Wasserstoff,

durch Umsetzung von 2,4-6-Trifluorpyrimidin (II)

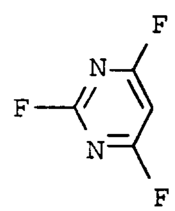

II

in einem aprotisch polaren organischen Lösungsmittel mit einem Amin der Formel III

$R^1$—$NH_2$ III

zum 2-Aminodifluorpyrimidinderivat der Formel IVa im Gemisch mit IVb,

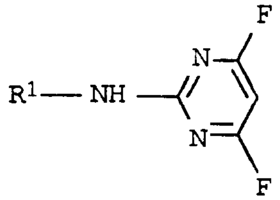

IVa

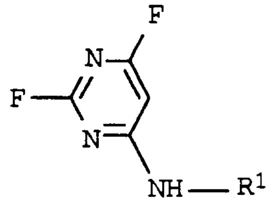

IVb

Abtrennung von IVa aus dem anfallenden Reaktionsgemisch und anschließenden Umsetzung von IVa mit einem Alkohol der Formel V

$R^2—OH$ V

in Gegenwart einer Base bei Temperaturen von 0 bis 180°C zum 2-Amino-4-fluorpyrimidinderivat der Formel I, oder durch Umsetzung von 2,4,6-Trifluorpyrimidin II in einem inerten organischen Lösungsmittel mit einem Alkohol der Formel V zum 2,4-Difluorpyrimidinether VIa im Gemisch mit dem 4,6-Difluorpyrimidinether VIb,

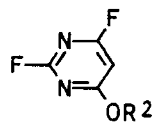
VIa

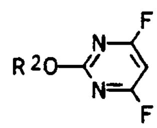
VIb

Abtrennung von VIa aus dem anfallenden Reaktionsgemisch und anschließende Umsetzung von VIa in einem aprotisch polaren Lösungsmittel mit einem Amin der Formel III zum 2-Amino-4-fluorpyrimidinderivat I.

Aus der Literatur (J.Med.Chem. 6, 688 (1963)) ist die Umsetzung von 2,4,6-Trifluorpyrimidin (II) mit Ammoniak zum Gemisch aus 2-Amino-4,6-difluorpyrimidin IVa' und 4-Amino-2,6-difluorpyrimidin IVb', die Isolierung von IVa' bzw. IVb' sowie die Umsetzung des Isomeren IVa' mit Methanol, Ethanol und Benzylalkohol V' zu den 2-Amino-4-fluorpyrimidin-derivaten I' bekannt.

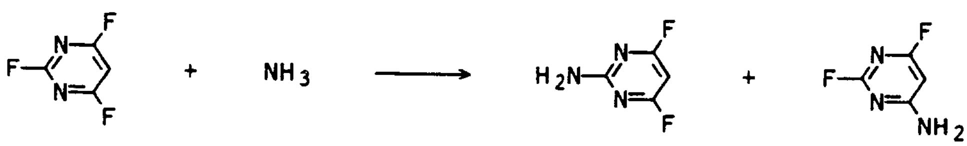

II III' IVa' IVb'

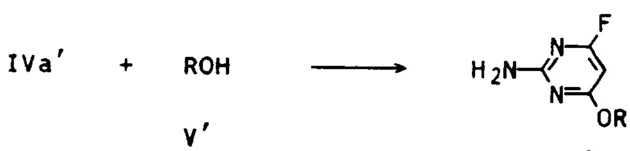

V' I'

(R = $CH_3$, $CH_2CH_3$, $CH_2Ph$)

Hierbei wurde in der ersten Stufe der Synthese absolutes Ethanol bei 0°C mit Ammoniak gesättigt, und die so erhaltene Lösung wurde sodann mit einer Lösung aus 2,4,6-Trifluorpyrimidin (II) und Ethanol versetzt. Nach aufwendiger Trennung der Isomeren konnte das 2-Amino-4,6-difluorpyrimidin IVa' in 54,9 % Ausbeute isoliert werden.

Die weitere Umsetzung des so erhaltenen 2-Aminodifluorpyrimidins IVa' mit den Alkoholen V' wurde mit den entsprechenden Natriumsalzen im Alkohol selbst (für Methanol und Ethanol) bzw. in Toluol (für Benzylalkohol) bei Temperaturen zwischen 55 und 110°C durchgeführt und lieferte das gewünschte 2-Amino-4-fluorpyrimidinderivat in 90 % d.Th. (R = $CH_3$), 61 % d.Th. (R = $CH_2CH_3$) bzw. 64 % d.Th. (R = $CH_2Ph$) Rohausbeute.

Daneben sind in der Literatur zwei weitere Synthesemethoden für das 2-Amino-4,6-difluorpyrimidin (IVa') beschrieben.

Banks et al (J.Chem.Soc. [C] 1280 (1970)) setzten 2,4,6-Trifluorpyrimidin (II) bei 0 bis 25°C mit einer gesättigten Lösung von Ammoniak in Tetrahydrofuran um und erhielten das Isomerengemisch aus IVa' und IVb' in 79 %iger Ausbeute im Verhältnis 67:33.

In der SU-A 547 447 (1975) wurde diese Umsetzung in Diethylether bei (-10)°C bis 25°C beschrieben, wobei die Isomeren IVa' und IVb' in 99 % Gesamtarnsbeute im Verhältnis 85:15 anfielen. Die dort ebenfalls beschriebene Umsetzung von IVa' mit Natriumbenzylat erfolgt analog den bereits zitierten Bedingungen (J.Med.Chem.).

Das wesentliche Problem dieser Synthesen für 2-Amino-4-fluorpyrimidin-derivate I ist die Reindarstellung von IVa. Zusätzlich liefert auch die Umsetzung von IVa mit dem Natriumalkoholat nur rohe Produkte, aus denen die gewünschten Derivate nur unter Verlust an Ausbeute isoliert werden können.

Der Erfindung lag daher die Aufgabe zugrunde, ein einfacheres und auch für den industriellen Maßstab wirtschaftlicheres Verfahren zu entwickeln, welches eine leichtere und effektivere Aufarbeitung gestattet.

Entsprechend dieser Aufgabe wurde ein Verfahren zur Herstellung der eingangs definierten 2-Amino-4-fluorpyrimidinderivate der Formel I durch Umsetzung von 2,4,6-Trifluorpyrimidin (II) in einem aprotisch polaren organischen Lösungsmittel mit einem Amin der Formel III

$R^1\text{-}NH_2$ III

zum 2-Aminodifluorpyrimidinderivat IVa im Gemisch mit IVb,

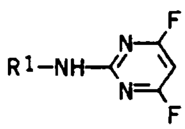
IVa

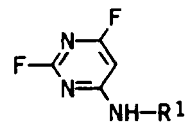
IVb

Abtrennung von IVa aus dem anfallenden Reaktionsgemisch und anschließende Umsetzung von IVa mit einem Alkohol der Formel V

$R^2OH$ (V)

In Gegenwart einer Base bei Temperaturen von 0 bis 180 °C gefunden, das dadurch gekennzeichnet ist, daß man die Umsetzung des 2,4,6-Trifluorpyrimidins bei einer Temperatur von (-80) °C bis (-15) °C durchführt und das 2-Aminodifluorpyrimidinderivat IVa mit dem Alkohol V in Gegenwart einer organischen Base oder mit einem Alkoholat Va in Gegenwart des entsprechenden Alkohols V oder eines Kationen komplexierenden Lösungsmittels zum 2-Amino-4-fluorpyrimidinderivat I umsetzt.

Daneben kann man die 2-Amino-4-fluorpyrimidinderivate I aber auch dadurch erhalten, daß man 2,4,6-Trifluorpyrimidin (II) zuerst bei -40 °C bis 120 °C mit dem Alkohol V im Alkohol als Lösungsmittel oder in einem Kationen komplexierenden Lösungsmittel in Abwesenheit einer Base umsetzt und nach der Isolierung den so erhaltenen Difluorpyrimidinether VIa bei -40 °C bis 100 °C mit dem Amin III zum 2-Amino-4-fluorpyrimidinderivat I umsetzt.

Beide Wege sind im nachstehenden Reaktionsschema wiedergegeben:

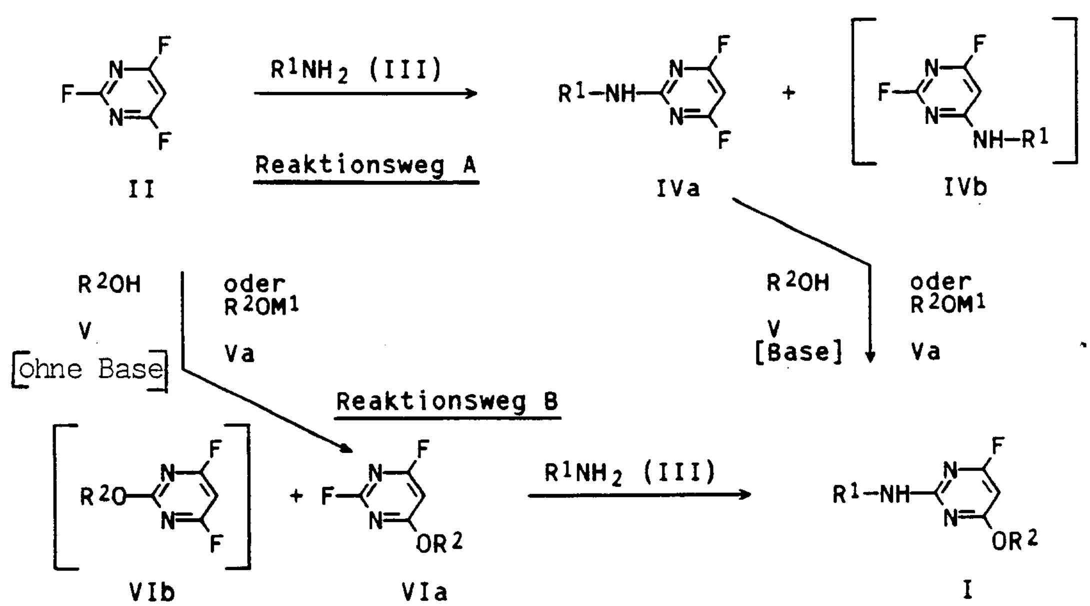

$M^1$ in Formel Va bedeutet ein Alkalimetallkation wie ein Lithium-, Natrium- und Kaliumkation oder das Äquivalent eines Erdalkalimetallkations wie ein Magnesium-, ein Calcium- und ein Bariumkation.

Die Herstellung der Verbindungen I erfolgt also auf zwei unabhängigen Reaktionswegen (A und B).

Reaktionsweg A

2,4,6-Trifluorpyrimidin II kann in einem aprotisch-polaren Lösungsmittel mit einem Amin III, welches in wäßriger Lösung vorliegen kann, bei einer Temperatur von -80 bis -15 °C umgesetzt werden, wonach das so erhaltene und isolierte 2-Aminopyrimidinderivat IVa ohne Lösungsmittel oder in Anwesenheit eines inerten organischen Lösungsmittels entweder mit einem Alkohol V in Anwesenheit einer organischen Base oder mit einem Alkoholat Va in Gegenwart des entsprechenden Alkohols V oder eines Kationen komplexierenden Lösungsmittels bei Temperaturen zwischen 0 und 180 °C zum 2-Amino-4-fluorpyrimidinderivat I umgesetzt wird. Diese Umsetzungen können bei Normaldruck oder unter Druck (1 bis 10 bar, vorzugsweise 1 bis 5 bar), kontinuierlich oder diskontinuierlich durchgeführt werden.

Für die Umsetzung des 2,4,6-Trifluorpyrimidins II mit dem Amin $R^1NH_2$ III zu IVa bzw. IVb sind folgende Lösungsmittel geeignet:
Ether wie Methyl-tert.-butylether, Diethylether, Ethylpropylether, n-Butylethylether, Di-n-butylether, Diisobutylether, Diisoamylether, Diisopropylether, Cyclohexylmethylether, Tetrahydrofuran, 1,2-Dimethoxyethan, Diethylenglycoldimethylether und Anisol, Ester wie Ethylacetat, n-Butylacetat und iso-Butylacetat sowie chlorierte Kohlenwasserstoffe wie 1,1,2,2-Tetrachlorethan 1,1-Dichlorethylen, Chlorbenzol, 1,2-Dichlorbenzol und 1-Chlornaphthalin und Gemische dieser Lösungsmitteln.

Besonders vorteilhaft verwendet man bei der Umsetzung von II mit III als Lösungsmittel einen Ether, Ester oder Halogenkohlenwasserstoff mit einer Lösungsmittelpolarität $E_T^N$ unterhalb 0,35.

Zweckmäßigerweise verwendet man das Lösungsmittel in einer Menge von 100 bis 2000 Gew.%, vorzugsweise 500 bis 1500 Gew.%, bezogen auf den Ausgangsstoff II.

Das Amin III wird vorteilhaft in Mengen von 1,8 bis 2,2 mol-Äquivalenten, bezogen auf II, eingesetzt. Besonders vorteilhaft gibt man 1,85 bis 2,15 mol-Äquivalent des Amins III, bezogen auf den Ausgangsstoff II innerhalb 1 bis 2 Stunden zu einer Mischung von Ausgangsstoff II in einem der vorgenannten Lösungsmittel bei (-80) bis (-15) °C, vorzugsweise (-30) bis (-15) °C, rührt zur Vervollständigung der Reaktion bis zu einer Stunde nach und läßt dann zur Aufarbeitung auf 25 °C erwärmen.

Die Umsetzung der Zwischenstufe IVa mit dem Alkohol V führt man zweckmäßigerweise direkt in überschussigem Alkohol V als Lösungsmittel durch. Bei Verwendung einer organischen Base eignet sich insbesondere ein tertiäres Amin, das z.B. in Mengen von 0,9 bis 1,2 mol-Äquivalenten, bezogen auf IVa, zugesetzt werden kann. Zur Reaktionsbeschleunigung kann es besonders von Vorteil sein, als Base ein Alkoholat Va zuzusetzen. Im allgemeinen wird dieses Alkoholat zu einer Suspension des Ausgangsstoffes IVa in der 5-bis 30-fachen Gewichtsmenge Alkohol V als Lösungsmittel, bezogen auf den Ausgangsstoff IVa, innerhalb einer Stunde bei einer Temperatur von 20 bis 80 °C gegeben. Zur Beendigung der Umsetzung rührt man dann noch 0,5 bis 8 Stunden bei 0 bis 140 °C, vorzugsweise 20 bis 100 °C.

Als Alkoholate Va kommen beispielsweise Lithium-, Natrium-, Kalium-, Calcium-, Barium- und Magnesiumsalze in Betracht. Es kommen anstelle der Alkoholate jedoch auch organische Basen wie Trimethylamin, Triethylamin, N-Ethyldiisopropylamin, Triisopropylamin, N,N-Dimethylamin, N,N-Dimethylcyclohexylamin, N-Methylpyrrolidin, Pyridin, Chinolin, $\alpha,\beta,\gamma$-Picolin, 2,4- und 2,6-Lutidin und Triethylendiamin zur Reaktionsbeschleunigung in Betracht.

Nach einer besonderen Ausgestaltung des erfindungsgemäßen Verfahrens kann man das 2-Aminodifluorpyrimidinderivat IVa in einem organischen Lösungsmittel mit einem Alkoholat Va umsetzen, welches die Kationen komplexiert.

Derartige komplexierende Lösungsmittel sind beispielsweise Dimethylsulfoxid, Dimethylformamid und N-Methyl-2-pyrrolidon. Üblicherweise werden diese Lösungsmittel in 5 mol-Äquivalent bis 15 mol-Äquivalent, bezogen auf IVa und das Alkoholat Va in 0,9 mol-Äquivalent bis 1,2 mol-Äquivalent, bezogen auf IVa, eingesetzt. Die Lösungsmittelpolarität des komplexierenden Lösungsmittels $E_T^N$ liegt vorteilhaft im Bereich von 0,5 bis 0,3.

Diese Umsetzung kann vorzugsweise bei Temperaturen von 60 bis 180 °C, vorzugsweise 80 bis 150 °C, kontinuierlich oder diskontinuierlich durchgeführt werden.

Reaktionsweg B

Sofern man 2,4,6-Trifluorpyrimidin in der ersten Reaktionsstufe mit einem Alkohol V verethert und den so gebildeten Pyrimidinether VIa anschließend mit dem Amin III in das 2-Amino-4-fluorpyrimidinderivat I

überführt, verlaufen diese Umsetzungen analog zu den vorstehend geschilderten Bedingungen in den gleichen Lösungsmitteln bei Verwendung gleicher Mengenverhältnisse. Erfindungsgemäß wird die Umsetzung des 2,4,6-Trifluorpyrimidins (II) mit dem Alkohol V in Abwesenheit einer Base durchgeführt.

Die Reaktionstemperatur liegt bei der Umsetzung von II mit dem Alkohol V bzw. seinem Salz Va zwischen -40 und 120 °C, vorzugsweise zwischen -20 und 100 °C, und bei der Umsetzung von VIa mit einem Amin III zwischen -40 und 100 °C, vorzugsweise zwischen -20 und +40 °C.

Die Reaktionszeiten betragen bei beiden Umsetzungen bis zu 6 Stunden.

Zur Isolierung der Aminopyrimidine I dienen die hierfür in der Literatur üblichen Aufarbeitungsmethoden.

Der Vorteil des Reaktionsweges A gegenüber dem Stand der Technik liegt in dem raschen und direkten Zugang zu dem isomerenfreien 2-Amino-4,6-difluorpyrimidin IVa in einer Hauptreaktion ohne zusätzliche Wasch- und Umkristallisationsprozesse sowie ohne verlustreiche Wasserdampfdestillation. Auch die folgende Umsetzung mit Alkoholen liefert direkt reine 6-Alkoxy-2-amino-4-fluorpyrimidine I.

Der Vorteil des Reaktionsweges B gegenüber dem Stand der Technik liegt in der höheren Gesamtausbeute der erhaltenen Alkoxypyrimidine VIa und VIb [gegenüber J.Chem.Soc. 6, 1280 (1970)], ihrer leichten destillativen Trennung [ohne verlustreiche Wasserdampfdestillation gegenüber J. Med. Chem. 6, 688 (1963)], ferner in der basenfreien Umsetzung (Beispiel 15) im ersten Reaktionsschritt sowie in der isomerenfreien Aminierung zu I.

In den Ausgangsstoffen III steht der Rest $R^1$ für folgende Gruppen:

Wasserstoff;

Alkyl, ausgewählt aus der Gruppe Methyl,

Ethyl, Propyl, 1-Methylethyl, n-Butyl, 1-Methylpropyl, 2-Methylpropyl und 1,1-Dimethylethyl sowie n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 2-Methylhexyl, 3-Methylhexyl, 4-Methylhexyl, 5-Methylhexyl, 1-Ethylpentyl, 2-Ethylpentyl, 1-Propylbutyl und Octyl, vorzugsweise Methyl, Ethyl, Propyl, 1-Methylethyl und 1,1-Dimethylethyl;

Alkenyl, ausgewählt aus der Gruppe Allyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1,2-Dimethyl-2-propenyl, 1,1-Dimethyl-2-propenyl, 1-Methyl-3-butenyl, 1-Ethyl-2-propenyl, 2-Hexenyl, 1-Methyl-2-pentenyl, 1-Ethyl-2-butenyl und 2-Ethyl-2-butenyl, vorzugsweise Allyl, 2-Butenyl und 3-Butenyl;

Alkinyl, ausgewählt aus der Gruppe 2-Propinyl, 2-Butinyl, 3-Butinyl, 1-Methyl-2-propinyl, 1-Methyl-2-butinyl, 1-Ethyl-2-propinyl, 2-Pentinyl, 3-Pentinyl, 4-Pentinyl und 1-Methyl-2-pentinyl, bevorzugt 2-Propinyl;

Cycloalkyl wie Cyclopropan, Cyclobutan, Cyclopentan, Cyclohexan, Cycloheptan und Cyclooctan, bevorzugt der Cyclopropan, Cyclobutan, Cycloheptan, Cyclohexan und Cycloheptan;

Phenyl oder Benzyl.

In den Ausgangsstoffen V bzw. Va bedeutet der Rest $R^2$ einen der für $R^1$ genannten Reste, jedoch nicht Wasserstoff.

Im Hinblick auf den Stand der Technik liefert das erfindungsgemäße Verfahren in allen Schritten auf einfacherem und wirtschaftlicherem Weg die Verbindungen I in vergleichsweise besserer Ausbeute und Reinheit.

Die so erfindungsgemäß herstellbaren 2-Amino-4-fluorpyrimidinderivate der Formel I sind wertvolle Ausgangsstoffe für Farbstoffe, Pharmazeutika und Pflanzenschutzmittel.

Beispiel 1

Herstellung von 2-Amino-4-fluor-6-methoxypyrimidin (Variante A)

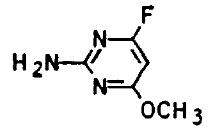

a) 2-Amino-4,6-difluorpyrimidin

69,7 g (4,1 mol) flüssiges Ammoniak wurden bei -30 bis -20 °C innerhalb 1 Stunde unter Rühren zu einer

Mischung aus 250 g (1,865 mol) 2,4,6-Trifluorpyrimidin und 3,3 l Diethylether gegeben. Zur Aufarbeitung erwärmte man das Reaktionsgemisch auf 25 °C und filtrierte den ausgefallenen Niederschlag ab. Nach dem Waschen mit Ether, Verrühren in Wasser, erneutem Filtrieren und Trocknen wurden 203 g (83 % d.Th.) des gewünschten Produkts erhalten (Fp. 214-216 °C).

Durch Einengen des Etherfiltrates auf ca. 1/3 seines Volumens konnten weitere 20 g (8 % d.Th.) dieser Verbindung vom Fp. 193-196 °C aus einem 1:1-Gemisch mit der isomeren 4-Aminoverbindung isoliert werden.

Vergleichsversuch (gemäß SU-A 547 447 - 1975)

Unter gleichen Reaktionsbedingungen wie a), jedoch bei einer Zugabe- und Reaktionstemperatur von (-10 °C) wurden aus 100 g (0,746 mol) 2,4,6-Trifluorpyrimidin und 29,1 g (1,71 mol) Ammoniak 76 g (78 % d.Th.) der Titelverbindung vom Fp. 212-213 °C und aus dem Filtrat weitere 19,5 g (20 % d.Th.) eines Gemisches (40:60) der gesuchten Verbindung und der isomeren 4-Aminoverbindung erhalten (Fp. 195-196 °C).

b) 2-Amino-4-fluor-6-methoxypyrimidin (Variante A)

27 g 30 %iges Natriummethylat (0,15 mol) wurden innerhalb von 20 Minuten unter Rühren bei 65 °C zu einer Suspension von 19,7 g (0,15 mol) 2-Amino-4,6-difluorpyrimidin in 250 ml abs. Methanol gegeben. Nach 5 Stunden Rühren unter Rückfluß wurde die Reaktionslösung auf 25 °C abgekühlt, der ausgefallene Niederschlag abgetrennt, mit wenig Methanol gewaschen und dann in Wasser verrührt. Nach dem Abfiltrieren, Waschen mit Wasser und Trocknen erhielt man 16 g (74 % d.Th.) der Titelverbindung vom Fp. 172 °C. Durch Einengen des Filtrats, Waschen mit Methanol und anschließend mit Wasser wurden weitere 3 g (14 % d. Th.) der Titelverbindung vom Fp. 161-169 °C isoliert.

Beispiel 2

Herstellung von 2-Amino-6-ethoxy-4-fluorpyrimidin (Variante A)

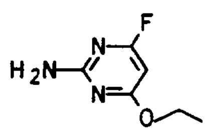

Eine Lösung von 25,2 g (0,3 mol) Kaliumethylat in 200 ml abs. Ethanol wurde innerhalb 40 Minuten unter Rühren bei 78 °C zu einer Suspension von 39,3 g (0,3 mol) 2-Amino-4,6-difluorpyrimidin (s. Beispiel 1a) in 150 ml abs. Ethanol gegeben. Nach 5 Stunden Rühren unter Rückfluß wurde abgekühlt, wonach das Lösungsmittel unter vermindertem Druck entfernt wurde. Der Rückstand wurde mit Wasser verrührt, abfiltriert, mit Wasser gewaschen und getrocknet. Man erhielt 39 g (83 % d.Th.) der Titelverbindung vom Fp. 121-123 °C (vgl. Lit. J. Med. Chem. 6, 688 (1963); 61 % Ausbeute an Rohprodukt; Fp nach Umkristallisation: 120,5-123 °C).

Beispiel 3

Herstellung von 2-Amino-4-fluor-6-propyloxypyrimidin (Variante A)

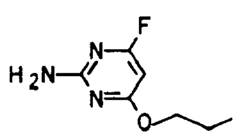

29,4 g (0,3 mol) Kaliumpropylat wurden analog Beispiel 1 mit 39,3 g (0,3 mol) 2-Amino-4,6-difluorpyrimidin in insgesamt 400 ml n-Propanol umgesetzt. Aus der Reaktionsmischung wurde das Lösungsmittel bei vermindertem Druck entfernt und der Rückstand wurde mit Petrolether gewaschen. Anschließend wurde in Wasser verrührt, abfiltriert, gewaschen und getrocknet, wobei 36,1 g (70 % d. Th.) der Titelverbindung vom Fp. 63-66 °C anfielen.

Beispiel 4

Herstellung von 2-Amino-4-fluor-6-isopropyloxypyrimidin (Variante A)

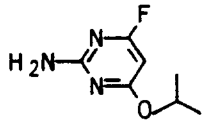

29,4 g (0,3 Mol) Kaliumisopropylat wurden analog zu 1.2 mit 39,3 g (0,3 Mol) 2-Amino-4,6-difluorpyrimidin (A.1a) in insgesamt 400 ml Isopropanol umgesetzt. Nach der Aufarbeitung, Waschen mit Petrolether und Wasser in üblicher Weise erhielt man 38,5 g (75 % d. Th.) der Titelverbindung vom Fp. 66-68 ° C.

Beispiel 5

Herstellung von 6-Allyloxy-2-amino-4-fluorpyrimidin (Variante A)

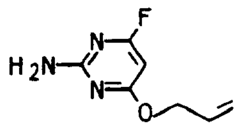

Man gab bei 25 ° C unter Stickstoff-Atmosphäre 1,14 g (0,0382 mol) 80 %iges Natriumhydrid (Emulsion in Leinöl) zu 70 ml Allylalkohol. Nach 20 Minuten Rühren bei 40 ° C wurde diese klare Lösung mit 5,0 g (0,0382 mol) 2-Amino-4,6-difluorpyrimidin versetzt und anschliepend 1,5 Stunden bei 97 ° C gerührt. Zur Aufarbeitung wurde der überschüssige Alkohol bei vermindertem Druck abdestilliert, der Rückstand in Methylenchlorid aufgenommen, mit Wasser gewaschen, über Magnesiumsulfat getrocknet und anschließend vom Lösungsmittel befreit. Das so erhaltene zähe öl kristallisierte beim Anreiben mit n-Pentan. Nach dem Abfiltrieren, Waschen mit Wasser und Trocknen erhielt man so 4,6 g (71,2 % d.Th.) der Titelverbindung vom Fp. 62-66 ° C.

Beispiel 6

Herstellung von 2-Amino-6-cyclohexyloxy-4-fluorpyrimidin (Variante A)

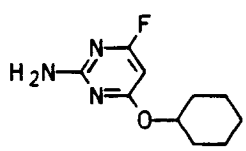

Unter Stickstoff wurden 1,14 g (0,0382 mol) 80 %iges Natriumhydrid bei 25 ° C zu 80 ml Cyclohexanol gegeben, wonach diese Mischung 2 Stunden bei 120 ° C gerührt wurde. Nach dem Abkühlen auf 40 ° C wurden mit 5,0 g (0,0382 mol) 2-Amino-4,6-difluorpyrimidin versetzt und anschließend 8 Stunden bei 110 ° C gerührt. Nach beendeter Umsetzung wurde das überschüssige Cyclohexanol bei vermindertem Druck abdestilliert und der so erhaltene Rückstand wurden 30 ml Methanol gelöst und durch Wasserzugabe zur Kristallisation gebracht. Nach dem Abfiltrieren, Waschen mit Wasser und Trocknen erhielt man 6,4 g (79,4 % d.Th.) der Titelverbindung vom Fp. 89-91 ° C.

Beispiel 7

Herstellung von 2-Amino-6-benzyloxy-4-fluorpyrimidin (Variante A)

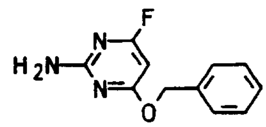

Unter Stickstoff wurden 1,14 g (0,0382 mol) 80 %iges Natriumhydrid bei 25 ° C zu 70 ml Benzylalkohol gegeben und diese Mischung 30 Minuten bei 100 ° C gerührt. Nach dem Abkühlen auf 40 ° C wurde mit 5,0 g (0,0382 mol) 2-Amino-4,6-difluorpyrimidin versetzt und anschließend 5 Stunden bei 100 ° C gerührt. Nach beendeter Umsetzung wurde von wenig Niederschlag abfiltriert und bei 150 ° C Badtemperatur bei 1 mbar eingeengt. Der so erhaltene zähe Rückstand wurde durch Anreiben mit Cyclohexan zur Kristallisation gebracht. Nach dem Abfiltrieren, Waschen mit Wasser und Trocknen erhielt man so 6,3 g (75,2 % d.Th.) der Titelverbindung vom Fp. 96-98 ° C.

Beispiel 8

Herstellung von 2-Amino-4-fluor-6-n-heptyloxypyrimidin (Variante A)

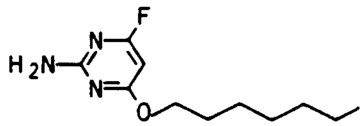

Zu einer Mischung von 5 g (0,038 mol) 2-Amino-4,6-difluorpyrimidin in 70 ml n-Heptanol gab man bei 20 bis 25 ° C innerhalb 5 Minuten unter Rühren eine Lösung von 1,14 g (0,038 mol) 80 %iges Natriumhydrid (Suspension in Leinöl) in 30 ml n-Heptanol. Nach 2,5 Stunden wurde analog Beispiel 6 aufgearbeitet. Man erhielt so 6,3 g (73 % d.Th.) der Titelverbindung vom Fp. 30-32 ° C.

Beispiel 9

Herstellung von 2-Amino-6-(4-chlorphenoxy)-4-fluorpyrimidin (Variante A)

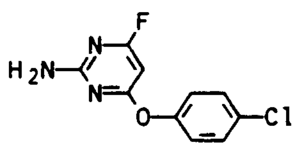

2.52 g (0,038 mol) 85 %iges Natriumhydroxid wurden in 50 ml Methanol gelöst, mit 4,9 g (0,0382 mol) 4-Chlorphenol versetzt und zur Trockene eingeengt. Der so erhaltene Salzrückstand wurde bei 25 ° C in 50 ml N-Methyl-2-pyrrolidon mit 5,0 g (0,0382 mol) 2-Amino-4,6-difluorpyrimidinversetzt und 4 Stunden bei 140 ° C gerührt. Nach dem Abkühlen auf 25 ° C wurde die Reaktionsmischung in 500 ml Wasser eingerührt und der entstandene Niederschl)g isoliert. Man erhielt so 7,4 g (81 % d.Th.) der Titelverbindung vom Fp. 223-226 ° C.

Beispiel 10

Herstellung von 4,6-Difluor-2-n-propylaminopyrimidin (Variante A)

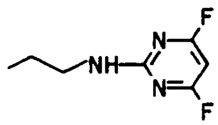

Zu einer Mischung aus 13,4 g (0,1 mol) 2,4,6-Trifluorpyrimidin in 150 ml Diethylether wurden bei -20 ° C innerhalb 20 Minuten 13,0 g (0,22 mol) n-Propylamin gegeben. Nach 1 Stunde Rühren bei -20 ° C und 1 Stunde bei 25 ° C wurde vom Niederschlag abfiltriert, die organische Phase mit Wasser gewaschen, getrocknet und bei vermindertem Druck eingeengt. Man erhielt so 15,8 g (91,3 % d.Th.) der Titelverbindung als Öl ($n_D^{23}$ = 1.4965).

Beispiel 11

Herstellung von 2-Allylamino-4,6-difluorpyrimidin (Variante A)

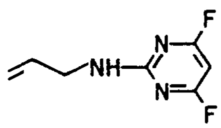

Aus 12,5 g (0,22 mol) Allylamin und 13,4 g (0,1 mol) 2,4,6-Trifluorpyrimidin in 150 ml Ether erhielt man analog Beispiel 10 11,1 g (65 % d.Th.) der Titelverbindung vom Fp. 52-54 ° C.

Beispiel 12

Herstellung von 2-Benzylamino-4,6-difluorpyrimidin (Variante A)

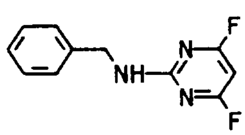

23,5 g (0,22 mol) Benzylamin wurden innerhalb 15 min unter Rühren bei -20 ° C zu einer Mischung von 13,4 g (0,1 mol) 2,4,6-Trifluorpyrimidin in 150 ml Diethylether gegeben und 1 Stunde bei dieser Temperatur gerührt. Nach einer weiteren Stunde bei 25 ° C wurde analog Beispiel 10 aufgearbeitet. Man erhielt so 21,4 g (98 % d.Th.) der Titelverbindung vom Fp. 70-73 ° C.

Beispiel 13 (Teilschritt a) nicht erfindungsgemäß)

Herstellung von 2-Amino-6-fluor-4-methoxypyrimidin (Variante B)

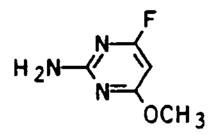

a) 2,4-Difluor-6-methoxypyrimidin

Zu einer Mischung aus 250 g (1,865 mol) 2,4,6-Trifluorpyrimidin in 1,4 l Methanol wurden bei -20 °C innerhalb von 45 Minuten 335,8 g (1,865 mol) 30 %iges Natriummethylat (in Methanol) gegeben und weitere 30 Minuten bei dieser Temperatur gerührt. Anschließend ließ man auf 25 °C erwärmen und engte die Reaktionsmischung auf ca. 1/5 ihres Volumens ein.

Das so erhaltene Gemisch wurde zwischen Diethylether und Wasser verteilt, wonach die organische Phase über Magnesiumsulfat getrocknet und eingeengt wurde. Nach Destillation (1,1 m Kolonne, 3 mm V-Füllkörper) erhielt man 141,6 g (52 % d.Th.) der Titelverbindung von Kp.: 144-145 °C.

Aus dem Destillationssumpf erhielt man durch Destillation über einen Normag-Aufsatz 114,4 g (42 % d.Th.) an 4,6-Difluor-2-methoxypyrimidin vom Kp.: 157-161 °C.

b) 2-Amino-6-fluor-4-methoxypyrimidin

13,6 g (0,8 mol) Ammoniak in 30 ml Methyl-tert.-butylether wurden bei -20 bis -10 °C innerhalb 20 Minuten unter Rühren zu einer Mischung von 52 g (0,356 mol) 2,4-Difluor-6-methoxypyrimidin in 300 ml Methyl-tert.-butylether gegeben. Nach weiteren 2 Stunden bei -15 °C und 3 Stunden bei 25 °C wurde der ausgefallene Niederschlag abgesaugt, mit Methyl-tert.-butylether gewaschen, mit Wasser verrührt, abfiltriert, erneut gewaschen und anschließend getrocknet, wobei 36,1 g (71 % d. Th.) der Titelverbindung vom Fp. 171-173 °C anfielen.

Beispiel 14

Herstellung von 4-Fluor-6-methoxy-2-methylaminopyrimidin (Variante B)

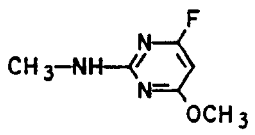

46,8 g einer 40 %igen Methylaminlösung in Wasser (0,605 mol) wurden bei 0 bis 2 °C innerhalb 30 Minuten unter Rühren zu einer Mischung von 41,9 g (0,287 mol) 2,4-Difluor-6-methoxypyrimidin (Beispiel 13a) und einer Spatelspitze Triethyl-benzylammoniumchlorid (Phasentransferkatalysator) in 100 ml Methyl-tert.-butylether gegeben. Nach 1 Stunde bei 0 °C und 3 Stunden bei 25 °C wurde die organische Phase abgetrennt, mit Wasser gewaschen und bei vermindertem Druck eingeengt. Der Rückstand wurde mit Pentan verrührt, wobei 39,9 g (88 % d. Th.) 4-Fluor-6-methoxy-2-methyl-amino-pyrimidin vom Fp. 78-80 °C anfielen.

Beispiel 15

Herstellung von 2,4-Difluor-6-methoxypyrimidin (Variante B)

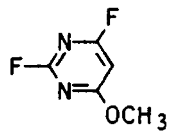

Unter Stickstoffatmosphäre wurden 250 g (1,865 mol) 2, 4, 6-Trifluorpyrimidin 4 Stunden in 2,5 l Methanol zum Sieden erhitzt und anschließend destilliert (bei längerer Reaktionszeit Dimethyletherbildung sowie Zersetzung).

| | |
|---|---|
| Fraktion I | (Normag-Aufsatz): 70,0 g (28 % d.Th.) unumgesetztes 2,4,6-Trifluorpyrimidin vom Kp: 104-110 °C |
| Fraktion II | (Füllkörperkolonne wie Beispiel 13a) 111,6 g (41 % d.Th.) der Titelverbindung vom Kp.: 144-145 °C |
| Fraktion III | (Füllkörperkolonne wie Beispiel 13a) 24,5 g (9 % d.Th.) 4,6-Difluor-2-methoxypyrimidin vom Kp: 157-161 °C |

Auf den Umsatz von 72 % bezogen auf 2,4,6-Trifluorpyrimidin ergibt sich daraus eine Ausbeute von 57 % d.Th. an der Titelverbindung und 12,5 % d.Th. an der 2-Methoxyverbindung.

Beispiel 16

Herstellung von 2-Cyclohexylamino-4-fluor-6-methoxypyrimidin (Variante B)

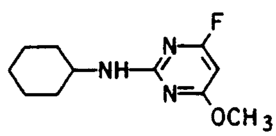

Zu einer Mischung aus 7,3 g (0,05 mol) 2,4-Difluor-6-methoxypyrimidin in 50 ml Methyl-t.-butylether wurden innerhalb 15 Minuten bei 0 bis 2 °C 10,1 g (0,102 mol) Cyclohexylamin gegeben. Nach 1 Stunde bei 0 °C und 3 Stunden bei 25 °C wurde der entstandene Niederschlag abfiltriert und das Filtrat mit Wasser gewaschen, über Magnesiumsulfat getrocknet und eingeengt. Aus dem so erhaltenen Rückstand isolierte man nach Chromatographie 8,3 g (73,8 % d.Th.) der Titelverbindung als Öl ($n_D^{23}$ = 1.5211).

Beispiel 17

Herstellung von 4-Fluor-2-n-hexylamino-6-methosypyrimidin (Variante B)

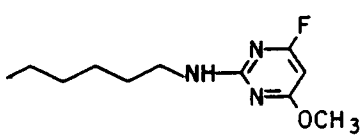

15,6 g (0,154 mol) n-Hexylamin wurden innerhalb 15 Minuten unter Rühren bei 0 bis 2 °C zu einer Mischung von 10,4 g (0,071 mol) 2,4-Difluor-6-methoxypyrimidin in 60 ml Methyl-t.-butylether gegeben. Nach 1 Stunde bei 0 °C, 4 Stunden bei 25 °C und Aufarbeitung analog Beispiel 16 erhielt man 9,6 g (60 % d.Th.) der Titelverbindung vom $n_D^{23}$ = 1.4938.

## Patentansprüche

1. Verfahren zur Herstellung von 2-Amino-4-fluorpyrimidinderivaten der allgemeinen Formel I

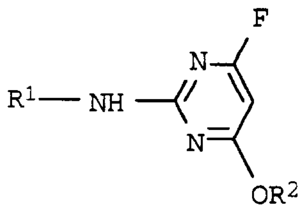

I

in der die Reste folgende Bedeutung haben:

$R^1$ Wasserstoff, Alkyl, ausgewählt aus der Gruppe Methyl, Ethyl, Propyl, 1-Methylethyl, n-Butyl, 1-Methylpropyl, 2-Methylpropyl und 1,1-Dimethylethyl sowie n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 2-Methylhexyl, 3-Methylhexyl, 4-Methylhexyl, 5-Methylhexyl, 1-Ethylpentyl, 2-Ethylpentyl, 1-Propylbutyl und Octyl, Alkenyl, ausgewählt aus der Gruppe Allyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1,2-Dimethyl-2-propenyl, 1,1-Dimethyl-2-propenyl, 1-Methyl-3-butenyl, 1-Ethyl-2-propenyl, 2-Hexenyl, 1-Methyl-2-pentenyl, 1-Ethyl-2-butenyl und 2-Ethyl-2-butenyl, Alkinyl, ausgewählt aus der Gruppe 2-Propinyl, 2-Butinyl, 3-Butinyl, 1-Methyl-2-propinyl, 1-Methyl-2-butinyl, 1-Ethyl-2-propinyl, 2-Pentinyl, 3-Pentinyl, 4-Pentinyl und 1-Methyl-2-pentinyl, $C_3$-$C_8$-Cycloalkyl, ein Phenylrest oder ein Benzylrest,

$R^2$ einer der Reste $R^1$ mit Ausnahme von Wasserstoff,

durch Umsetzung von 2,4,6-Trifluorpyrimidin (II)

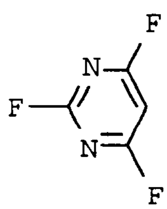

II

in einem aprotisch polaren organischen Lösungsmittel mit einem Amin der Formel III

$R^1$—$NH_2$ III

zum 2-Aminodifluorpyrimidinderivat der Formel IVa im Gemisch mit IVb,

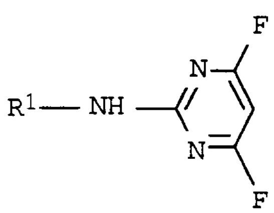

IVa

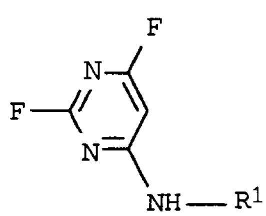

IVb

Abtrennung von IVa aus dem anfallenden Reaktionsgemisch und anschließenden Umsetzung von IVa mit einem Alkohol der Formel V

$R^2$—OH V

in Gegenwart einer Base bei Temperaturen von 0 bis 180 °C zum 2-Amino-4-fluorpyrimidinderivat I, welches dadurch gekennzeichnet ist, daß man die Umsetzung des 2,4,6-Trifluorpyrimidins II mit dem Amin III bei einer Temperatur von (-80) °C bis (-15) °C durchführt und das 2-Aminodifluorpyrimidinderivat IVa mit dem Alkohol V in Gegenwart einer organischen Base oder mit einem Alkoholat Va in Gegenwart des entsprechenden Alkohols V oder eines Kationen komplexierenden Lösungsmittels zum 2-Amino-4-fluorpyrimidinderivat I umsetzt.

**2.** Verfahren zur Herstellung von 2-Amino-4-fluorpyrimidinderivaten der allgemeinen Formel I gemäß Anspruch 1, durch Umsetzung von 2,4,6-Trifluorpyrimidin (II) in einem inerten organischen Lösungsmittel mit einem Alkohol der Formel V gemäß Anspruch 1, zum 2,4-Difluorpyrimidinether VIa im Gemisch mit dem 4,6-Difluorpyrimidinether VIb,

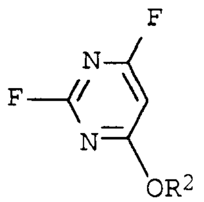
VIa

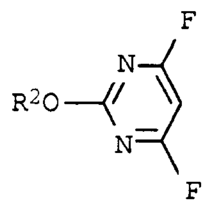
VIb

Abtrennung von VIa aus dem anfallenden Reaktionsgemisch und anschließende Umsetzung von VIa in einem aprotisch polaren Lösungsmittel mit einem Amin der Formel III gemäß Anspruch 1 zum 2-Amino-4-fluorpyrimidinderivat I, dadurch gekennzeichnet, daß man den Alkohol V im Alkohol als Lösungsmittel oder in einem Kationen komplexierenden Lösungsmittel in Abwesenheit einer Base umsetzt und die anschließende Umsetzung von VIa mit dem Amin III bei Temperaturen von -40 °C bis 100 °C durchführt.

**3.** Verfahren nach Anspruch 1, dadurch gekennzeichnet, daß man bei der Umsetzung von II mit III als Lösungsmittel einen Ether, Ester oder Halogenkohlenwasserstoff mit einer Lösungsmittelpolarität $E_T^N$ unterhalb 0,35 verwendet.

**4.** Verfahren nach den Ansprüchen 1 oder 2, dadurch gekennzeichnet, daß man das Amin III in 1,8 bis 2,2 mol-Äquivalenten, bezogen auf II, einsetzt.

**5.** Verfahren nach Anspruch 1, dadurch gekennzeichnet, daß man bei der Umsetzung des 2-Aminodifluorpyrimidins IVa mit dem Alkohol V als organische Base ein tertiäres Amin verwendet.

**6.** Verfahren nach Anspruch 5, dadurch gekennzeichnet, daß man das tertiäre Amin in 0,9 bis 1,2 mol-Äquivalenten bezogen auf IVa zusetzt.

**7.** Verfahren nach Anspruch 1, dadurch gekennzeichnet, daß man bei der Umsetzung des 2-Aminodifluorpyrimidins IVa mit dem Alkohol V als organische Base ein Alkalimetall- oder Erdalkalimetallsalz des entsprechenden Alkohols verwendet.

**8.** Verfahren nach Anspruch 7, dadurch gekennzeichnet, daß man das Alkalimetall- oder Erdalkalimetallalkoholat in 0,9 bis 1,2 mol-Äquivalenten bezogen auf IVa einsetzt.

**9.** Verfahren nach Anspruch 7, dadurch gekennzeichnet, daß man das 2-Aminodifluorpyrimidin IVa mit einem Alkalimetall- oder Erdalkalimetallsalz des Alkohols V in Gegenwart eines das Metallkation komplexierenden Lösungsmittels mit einer Lösungsmittelpolarität $E_T^N$ von 0,5 bis 0,3 umsetzt.

**10.** Verfahren nach Anspruch 9, dadurch gekennzeichnet, daß man das Alkalimetall- oder Erdalkalimetallsalz des Alkohols V in 0,9 bis 1,2 mol-Äquivalenten bezogen auf IVa einsetzt.

**11.** Verfahren nach Anspruch 10, dadurch gekennzeichnet, daß man das Lösungsmittel in 5 bis 15 mol-Äquivalenten bezogen auf IVa zusetzt.

**12.** Verfahren nach Anspruch 1 , dadurch gekennzeichnet, daß man eine Lösung des 2,4,6-Trifluorpyrimidins (II) mit dem Amin III versetzt.

**Claims**

**1.** A process for the preparation of a 2-amino-4-fluoropyrimidine derivative of the general formula I

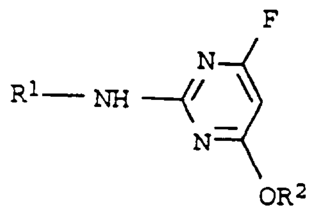

I

where $R^1$ is hydrogen, alkyl, selected from the group comprising methyl, ethyl, propyl, 1-methylethyl, n-butyl, 1-methylpropyl, 2-methylpropyl, 1,1-dimethylethyl, n-pentyl, 1-methylbutyl, 2-methylbutyl, 3-methylbutyl, 1,2-dimethylpropyl, 1,1-dimethylpropyl, 2,2-dimethylpropyl, 1-ethylpropyl, n-hexyl, 1-methylpentyl, 2-methylpentyl, 3-methylpentyl, 4-methylpentyl, 1,2-dimethylbutyl, 1,3-dimethylbutyl, 2,3-dimethylbutyl, 1,1-dimethylbutyl, 2,2-dimethylbutyl, 3,3-dimethylbutyl, 1,1,2-trimethylpropyl, 1,2,2-trimethylpropyl, 1-ethylbutyl, 2-ethylbutyl, 1-ethyl-2-methylpropyl, n-heptyl, 1-methylhexyl, 2-methylhexyl, 3-methylhexyl, 4-methylhexyl, 5-methylhexyl, 1-ethylpentyl, 2-ethylpentyl, 1-propylbutyl and octyl, alkenyl, selected from the group comprising allyl, 2-butenyl, 3-butenyl, 1-methyl-2-propenyl, 2-methyl-2-propenyl, 2-pentenyl, 3-pentenyl, 4-pentenyl, 1-methyl-2-butenyl, 2-methyl-2-butenyl, 3-methyl-2-butenyl, 1,2-dimethyl-2-propenyl, 1,1-dimethyl-2-propenyl, 1-methyl-3-butenyl, 1-ethyl-2-propenyl, 2-hexenyl, 1-methyl-2-pentenyl, 1-ethyl-2-butenyl and 2-ethyl-2-butenyl, alkynyl, selected from the group comprising 2-propynyl, 2-butynyl, 3-butynyl, 1-methyl-2-propynyl, 1-methyl-2-butynyl, 1-ethyl-2-propynyl, 2-pentynyl, 3-pentynyl, 4-pentynyl and 1-methyl-2-pentynyl, $C_3$-$C_8$-cycloalkyl, phenyl or benzyl,
$R^2$ is one of the $R^1$ radicals with the exception of hydrogen,
by reaction of 2,4,6-trifluoropyrimidine (II)

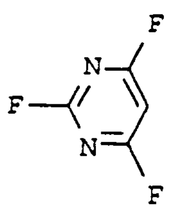

II

in an aprotic polar organic solvent with an amine of the formula III

$R^1$-$NH_2$ III

to give the 2-aminodifluoropyrimidine derivative of the formula IVa mixed with IVb

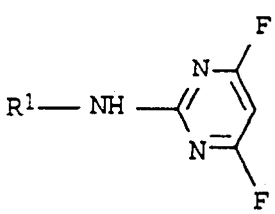

IVa

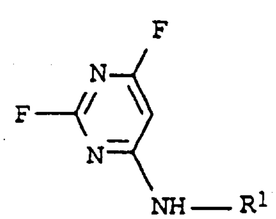

IVb

separation of IVa out of the resulting reaction mixture and subsequent reaction of IVa with an alcohol of

the formula V

$R^2$-OH V

in the presence of a base at from 0 to 180 °C to give the 2-amino-4-fluoropyrimidine derivative I, which comprises carrying out the reaction of 2,4,6-trifluoropyrimidine II with the amine III at from -80 °C to -15 °C, and reacting the 2-aminodifluoropyrimidine derivative IVa with the alcohol V in the presence of an organic base or with an alcoholate Va in the presence of the corresponding alcohol V or of a cation-complexing solvent to give the 2-amino-4-fluoropyrimidine derivative I.

**2.** A process for the preparation of a 2-amino-4-fluoropyrimidine derivative of the general formula I as claimed in claim 1, by reaction of 2,4,6-trifluoropyrimidine (II) in an inert organic solvent with an alcohol of the formula V as claimed in claim 1 to give the 2,4-difluoropyrimidine ether VIa mixed with the 4,6-difluoropyrimidine ether VIb,

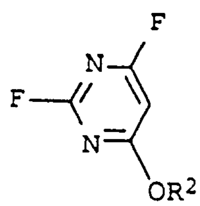 VIa 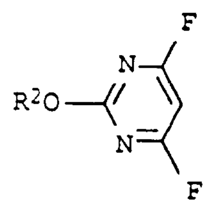 VIb

separation of VIa out of the resulting reaction mixture and subsequent reaction of VIa in an aprotic polar solvent with an amine of the formula III as claimed in claim 1 to give the 2-amino-4-fluoropyrimidine derivative I, which comprises reacting the alcohol V in the alcohol as the solvent or in a cation-complexing solvent in the absence of a base and carrying out the subsequent reaction of VIa with the amine III at from -40 °C to 100 °C.

**3.** A process as claimed in claim 1, wherein the solvent used in the reaction of II with III is an ether, ester or halogenated hydrocarbon with a solvent polarity $E_T^N$ below 0.35.

**4.** A process as claimed in claim 1 or 2, wherein the amine III is employed in 1.8 to 2.2 mole equivalents relative to II.

**5.** A process as claimed in claim 1, wherein a tertiary amine is used as organic base in the reaction of the 2-aminodifluoropyrimidine IVa with the alcohol V.

**6.** A process as claimed in claim 5, wherein the tertiary amine is added in 0.9 to 1.2 mole equivalents relative to IVa.

**7.** A process as claimed in claim 1, wherein an alkali metal or alkaline earth metal salt of the alcohol V is used as organic base in the reaction of the 2-aminodifluoropyrimidine IVa with the corresponding alcohol.

**8.** A process as claimed in claim 7, wherein the alkali metal or alkaline earth metal alcoholate is employed in 0.9 to 1.2 mole equivalents relative to IVa.

**9.** A process as claimed in claim 7, wherein the 2-aminodifluoropyrimidine IVa is reacted with an alkali metal or alkaline earth metal salt of the alcohol V in the presence of a solvent which complexes the metal cation and has a solvent polarity $E_T^N$ of 0.5 to 0.3.

**10.** A process as claimed in claim 9, wherein the alkali metal or alkaline earth metal salt of the alcohol V is employed in 0.9 to 1.2 mole equivalents relative to IVa.

**11.** A process as claimed in claim 10, wherein the solvent is added in 5 to 15 mole equivalents relative to IVa.

**12.** A process as claimed in claim 1, wherein the amine III is added to a solution of 2,4,6-trifluoropyrimidine (II).

**Revendications**

**1.** Procédé de préparation de dérivés de la 2-amino-4-fluoropyrimidine de la formule générale I

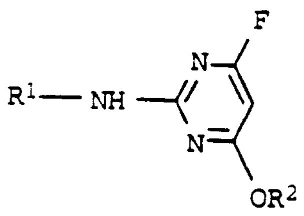

I

dans laquelle le symbole

$R^1$ représente un atome d'hydrogène, un radical alkyle choisi dans le groupe des radicaux qui suivent : méthyle, éthyle, propyle, 1-méthyléthyle, n-butyle, 1-méthylpropyle, 2-méthylpropyle et 1,1-diméthyléthyle, ainsi que n-pentyle, 1-méthylbutyle, 2-méthylbutyle, 3-méthylbutyle, 1,2-diméthylpropyle, 1,1-diméthylpropyle, 2,2-diméthylpropyle, 1-éthylpropyle, n-hexyle, 1-méthylpentyle, 2-méthylpentyle, 3-méthylpentyle, 4-méthylpentyle, 1,2-diméthylbutyle$_1$ 1,3-diméthylbutyle, 2,3-diméthylbutyle, 1,1-diméthylbutyle, 2,2-diméthylbutyle, 3,3-diméthylbutyle, 1,1,2-trimethylpropyle, 1,2,2-triméthylpropyle$_1$ 1-éthylbutyle, 2-éthylbutyle, 1-éthyl-2-méthylpropyle, n-heptyle, 1-méthylhexyle, 2-méthylhexyle, 3-méthylhexyle, 4-méthylhexyle, 5-méthylhexyle, 1-éthylpentyle, 2-éthylpentyle, 1-propylbutyle et octyle, un radical alcényle choisi dans le groupe formé par les radicaux qui suivent : allyle, 2-butényle, 3-butényle, 1-méthyl-2-propényle, 2-méthyl-2-propényle, 2-pentényle, 3-pentényle, 4-pentényle, 1-méthyl-2-butényle, 2-méthyl-2-butényle, 3-méthyl-2-butényle, 1,2-diméthyl-2-propényle, 1,1-diméthyl-2-propényle, 1-méthyl-3-butényle, 1-éthyl-2-propényle, 2-hexényle, 1-méthyl-2-pentényle, 1-éthyl-2-butényle et 2-éthyl-2-butényle, les radicaux alcynyle choisis dans le groupe formé par les radicaux qui suivent : 2-propynyle, 2-butynyle, 3-butynyle, 1-méthyl-2-propynyle, 1-méthyl-2-butynyle, 1-éthyl-2-propynyle, 2-pentynyle, 3-pentynyle, 4-pentynyle et 1-méthyl-2-pentynyle; les radicaux cycloalkyle en $C_3$ à $C_8$, un radical phényle ou un radical benzyle,

$R^2$ a les mêmes significations que $R^1$ à l'exception de l'hydrogène,

par la réaction de la 2,4,6-trifluoropyrimidine (II)

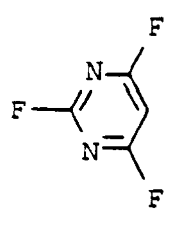

II

dans un solvant organique, polaire, aprotique, avec une amine de la formule III

$R^1—NH_2$ III

de manière à obtenir un dérivé de la 2-aminodifluoropyrimidine de la formule IVa en mélange à IVb,

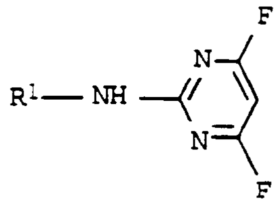 IVa 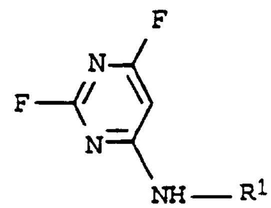 IVb

séparation de IVa du mélange réactionnel obtenu et réaction subséquente de IVa avec un alcool de la formule V

$R^2$—OH V

en présence d'une base, à des températures de 0 à 180 ° C, de manière à obtenir le dérivé de 2-amino-4-fluoropyrimidine I, procédé caractérisé en ce que l'on entreprend la réaction de la 2,4,6-trifluoropyrimidine II avec l'amine III à une température qui fluctue de -80 ° C à -15 ° C et on fait réagir le dérivé de la 2-aminodifluoropyrimidine IVa avec l'alcool V en présence d'une base organique, ou avec un alcoolate Va en présence de l'alcool V correspondant, ou d'un solvant complexant les cations, de manière à obtenir le dérivé de la 2-amino-4-fluoropyrimidine.

**2.** Procédé de préparation de dérivés de la 2-amino-4-fluoropyrimidine de la formule générale I suivant la revendication 1, par la réaction de la 2,4,6-trifluoropyrimidine (II) dans un solvant organique inerte avec un alcool de la formule V suivant la revendication 1, de manière à obtenir l'éther 2,4-difluoropyrimidinique VIa en mélange à l'éther 4,6-difluoropyrimidinique VIb,

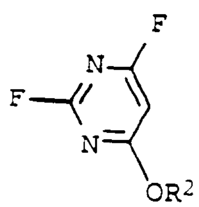 VIa 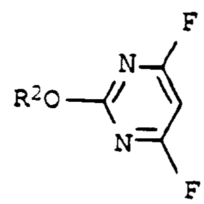 VIb

la séparation de VIa du mélange réactionnel obtenu et la réaction subséquente de VIa dans un solvant polaire et aprotique avec une amine de la formule III suivant la revendication 1, de manière à obtenir le dérivé de la 2-amino-4-fluoropyrimidine I, caractérisé en ce que l'on fait réagir l'alcool V dans l'alcool servant de solvant, ou dans un solvant complexant les cations, en l'absence d'une base et on entreprend la réaction subséquente de VIa avec l'amine III à des températures qui varient de -40 ° C à 100 ° C.

**3.** Procédé suivant la revendication 1, caractérisé en ce que, lors de la réaction de II avec III, on utilise un éther, un ester ou un hydrocarbure halogéné à titre de solvant, d'une polarité de solvant $E_T^N$ inférieure à 0,35.

**4.** Procédé suivant l'une quelconque des revendications 1 et 2, caractérisé en ce que l'on utilise l'amine III à raison de 1,8 à 2,2 équivalents molaires par rapport à II.

**5.** Procédé suivant la revendication 1, caractérisé en ce que, lors de la réaction de la 2-aminodifluoropyrimidine IVa avec l'alcool V, on utilise une amine tertiaire à titre de base organique.

**6.** Procédé suivant la revendication 5, caractérisé en ce que l'on ajoute l'amine tertiaire à raison de 0,9 à 1,2 équivalent molaire par rapport à IVa.

**7.** Procédé suivant la revendication 1, caractérisé en ce que, au cours de la réaction de la 2-aminodifluoropyrimidine IVa avec l'alcool V, on utilise un sel de métal alcalin ou de métal alcalino-terreux de l'alcool correspondant à titre de base organique.

8. Procédé suivant la revendication 7, caractérisé en ce que l'on utilise l'alcoolate de métal alcalin ou de métal alcalino-terreux à raison de 0,9 à 1,2 équivalent molaire par rapport à IVa.

9. Procédé suivant la revendication 7, caractérisé en ce que l'on fait réagir la 2-aminodifluoropyrimidine IV avec un sel de métal alcalin ou de métal alcalino-terreux de l'alcool V en présence d'un solvant complexant le cation de métal, d'une polarité de solvant $E_T^N$ de 0,5 à 0,3.

10. Procédé suivant la revendication 9, caractérisé en ce que l'on utilise le sel de métal alcalin ou de métal alcalino-terreux de l'alcool V à raison de 0,9 à 1,2 équivalent molaire par rapport à IVa.

11. Procédé suivant la revendication 10, caractérisé en ce que l'on ajoute le solvant à raison de 5 à 15 équivalents molaires par rapport à IVa.

12. Procédé suivant la revendication 1, caractérisé en ce que l'on ajoute l'amine III à une solution de la 2,4,6-trifluoropyrimidine (II).